# EUROPÄISCHE PATENTSCHRIFT

(11) **EP 0 780 362 B1**
(45) Veröffentlichungstag und Bekanntmachung des Hinweises auf die Patenterteilung: **20.10.1999**
(21) Anmeldenummer: 96119777.9
(22) Anmeldetag: 10.12.1996
(51) Int. Cl.: C07C 69/67, C07C 67/30, C07C 67/27, C07C 45/65, C07C 43/315, C07C 43/313

(54) **3,3-Dioxy-4,4,4-trifluorbuttersäure-Derivate**
3,3-Dioxy-4,4,4-trifluorobutyric acid derivatives
Dérivés de l'acide 3,3-dioxy-4,4,4-trifluorobutyrique

(30) Priorität: 22.12.1995 DE 19548416
(43) Veröffentlichungstag der Anmeldung: 25.06.1997
(73) Patentinhaber: BAYER AG, 51368 Leverkusen (DE)
(72) Erfinder: Lui, Norbert, Dr., 51061 Köln (DE)

(56) Entgegenhaltungen:
- GB-A- 931 689
- JOURNAL OF THE AMERICAN CHEMICAL SOCIETY, Bd. 75, Nr. 13, 5.Juli 1953, Seiten 3152-3153, XP000652734 MCBEE E.T. ET AL.: "The Preparation and Reactions of Fluorine-containing Acetoacetic Esters"
- DATABASE CROSSFIRE Beilstein BRN 1373760, XP002029001 & J. ORG. CHEM. USSR, Bd. 15, 1979, Seite 627 KONDRATENKO ET AL.: & J. FLUORINE CHEM., Bd. 57, 1992, Seiten 177-190, YAMANAKA ET AL.:
- DATABASE CROSSFIRE Beilstein BRN 1377272, XP002029002 & J. ORG. CHEM. USSR, Bd. 15, 1979, Seite 627 KONDRATENKO ET AL.: & J. FLUORINE CHEM., Bd. 44, 1989, Seiten 377-394, AUBERT ET AL.:
- DATABASE CROSSFIRE Beilstein BRN 1877961 XP002029003 & JUSTUS LIEBIG ANN. CHEM., Bd. 693, 1966, Seiten 134-157, GRELL & MACHLEIDT:
- DATABASE CROSSFIRE Beilstein BRN 5510882, XP002029004 & CHEM. LETT., 1988, Seiten 1987-1990, KUBOTA ET AL.:

## Beschreibung

Die Erfindung betrifft neue 3,3-Dioxy-4,4,4-trifluorbuttersäure-Derivate, ein Verfahren zu ihrer Herstellung und zur Herstellung anderer 3,3-Dioxy-4,4,4-trifluorbuttersäure-Derivate sowie neue Zwischenprodukte und Verfahren zu deren Herstellung.

Es ist bereits aus DE-OS 4025188 und J. Am. Chem. Soc. 72, 3289 (1950) bekannt, daß man 4,4,4-Trifluoracetessigester oder 2-Chlor-4,4,4-trifluoracetessigester unter sauren Bedingungen zu 1,1,1-Trifluoraceton oder 3-Chlor-1,1,1-trifluoraceton decarboxylieren kann. 1,1,1-Trifluoraceton und 1,1,1-Trifluor-3-halogenacetone sind wichtige Bausteine zur Herstellung von biologisch aktiven Verbindungen (siehe EP-625 516 A1, EP-491 137 A2).

Es wurden neue Verbindungen der Formel (I) gefunden 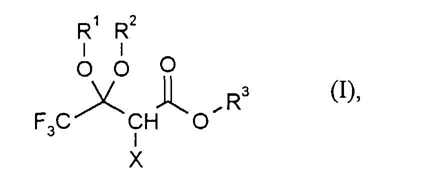 in welcher
- R¹ und R²: unabhängig voneinander für C₁-C₁₀-Alkyl, insbesondere Methyl, Ethyl, n-Propyl, Isopropyl, n-Butyl, Isobutyl, sec.-Butyl oder tert.-Butyl, für Cyclohexyl, für gegebenenfalls substituiertes C₆-C₁₀ -Aryl oder C₇-C₁₄-Arylalkyl stehen oder
- R¹ und R²: gemeinsam für gegebenenfalls ein- oder zweifach durch Methyl substituiertes C₂-C₄-Alkylen oder gegebenenfalls substituiertes ortho-Arylen stehen,
- R³: für Wasserstoff, C₁-C₁₀-Alkyl, insbesondere Methyl, Ethyl, n-Propyl, Isopropyl, n-Butyl, Isobutyl, sec.-Butyl, tert.-Butyl, Cyclohexyl, für gegebenenfalls substituiertes C₆-C₁₀-Aryl oder C₇-C₁₄-Arylalkyl steht und
- X: für Wasserstoff, Fluor, Chlor oder Brom steht,
wobei 4,4,4-Trifluor-3,3-dimethoxybuttersäuremethylester, 4,4,4-Trifluor-3,3-dimethoxybuttersäure, 4,4,4-Trifluor-3,3-diethoxybuttersäureethylester, 4,4,4-Trifluor-3,3-ethylendioxybuttersäureethylester und 4,4,4-Trifluor-3,3-ethylendioxybuttersäure ausgenommen sind, deren Herstellung auf grundsätzlich andere Weise bekannt ist.

Auf Verbindungen der Formel (I') wird im folgenden Bezug genommen, wenn die neuen Verbindungen der Formel (I) gemeinsam mit den vorgenannten bekannten Verbindungen gemeint sind.

Die Verbindungen der Formel (I), in welchen R³ ≠ Wasserstoff ist, lassen sich durch Einwirkung einer Base, z.B. durch wäßrige Alkali- oder Erdalkalimetallhydroxide oder -carbonate, und anschließendes Ansäuern zu den Säuren, d.h. Verbindungen der Formel (I), in welchen R³ = Wasserstoff ist, verseifen.

Weiterhin lassen sich die Verbindungen der Formel (I) durch Einwirken von wäßrigen Mineralsäuren bei Temperaturen von z.B. 50 bis 200°C, bevorzugt zwischen 90 und 150°C zu den bekannten 1,1,1-Trifluoracetonen der Formel (II) 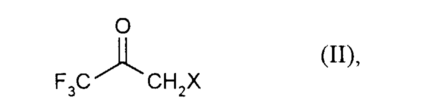 in der
- X: die bei Formel (I) angegebene Bedeutung hat,
umsetzen. Diese sind, wie oben erwähnt, interessante Bausteine für Heterocyclensynthesen.

Die Verbindungen der Formel (I) können durch Einwirkung von Lewissäuren, z.B. Bortribromid, in die entsprechenden 4,4,4-Trifluoracetessigesterderivate der Formel (III) 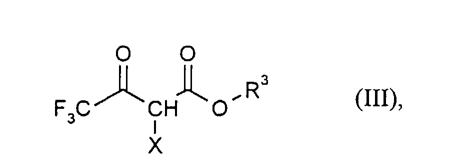 in der
- R³ und X: die bei Formel (I) angegebene Bedeutung haben,
überführt werden. Diese Verbindungen sind wichtige Zwischenprodukte für Fungizide (EP-371 950 A2).

In der Literatur beschrieben ist die Herstellung gegebenenfalls in 2-Position halogenierter 4,4,4-Trifluoracetessigester durch Kondensation von Estern gegebenenfalls halogenierter Essigsäuren oder deren Phosphorylide mit Trifluoressigsäureestern und gegebenenfalls anschließender Chlorierung oder Bromierung (vgl. z.B. J. Org. Chem. 56, 273 (1991); J. Fluorine Chem. 56, 271 (1992); J. Am. Chem. Soc. 69, 1819 (1947)). Bei der Ausführung der Esterkondensation gibt es zwei wesentliche Nachteile, die sich somit auch auf den bisher besten Syntheseweg zu den 1,1,1-Trifluoracetone auswirken. Zum einen sind die benötigten Trifluoressigsäureester nicht gut zugänglich und deshalb relativ teuer. Zum anderen werden bei Verwendung von technisch gängigen Basen wie z.B. Natriummethylat oder -ethylat nur mäßige Ausbeuten erreicht. Will man höhere Ausbeuten erreichen, müssen teure und insbesondere in der technischen Handhabung schwierige Basen wie Natriumhydrid oder Lithiumdiisopropylamid verwendet werden.

Diese Nachteile können durch die Verbindungen der Formel (I), welche durch das im folgenden beschriebene Verfahren gut zugänglich sind, überwunden werden.

Es wurde weiterhin gefunden, daß sich die Verbindungen der Formel (I') in einem Verfahren (A) herstellen lassen, das dadurch gekennzeichnet ist, daß man die neuen Verbindungen der Formel (IV) oder (V), 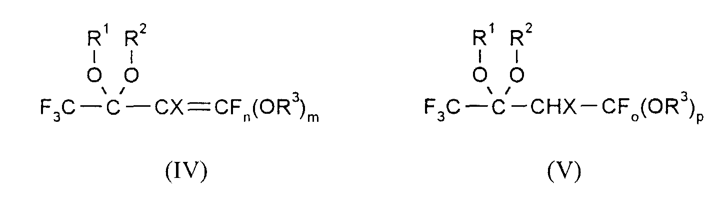 in welchen
- R¹ und R²: unabhängig voneinander für C₁-C₁₀-Alkyl, insbesondere Methyl, Ethyl, n-Propyl, Isopropyl, n-Butyl, Isobutyl, sec.-Butyl oder tert.-Butyl, für Cyclohexyl, für gegebenenfalls substituiertes C₆-C₁₀ -Aryl oder C₇-C₁₄-Arylalkyl stehen oder
- R¹ und R²: gemeinsam für gegebenenfalls ein- oder zweifach durch Methyl substituiertes C₂-C₄-Alkylen oder gegebenenfalls substituiertes ortho-Arylen steht,
- R³: für Wasserstoff, C₁-C₁₀-Alkyl, insbesondere Methyl, Ethyl, n-Propyl, Isopropyl, n-Butyl, Isobutyl, sec.-Butyl, tert.-Butyl, Cyclohexyl, für gegebenenfalls substituiertes C₆-C₁₀-Aryl oder C₇-C₁₄-Arylalkyl steht und
- X: für Wasserstoff, Fluor, Chlor oder Brom steht,
- n und o: unabhängig voneinander für die Zahlen 0, 1 oder 2 stehen,
- m: für die Zahlen 0, 1 oder 2 steht, wobei n+m = 2 ist und
- p: für 1, 2 oder 3 steht, wobei o+p = 3 ist,
mit einer Hydroxyverbindung der Formel (VI)

R³―OH (VI),

in der
- R³: die bei den Formeln (IV) und (V) angegebene Bedeutung hat,
in Gegenwart eines Protonenspenders umsetzt.

Es wurde weiterhin gefunden, daß sich die neuen Zwischenprodukte der Formel (IV) oder (V) in einem Verfahren (B) herstellen lassen, das dadurch gekennzeichnet ist, daß man die zum Teil neuen Dioxyhexafluorbutane der Formel (VII) 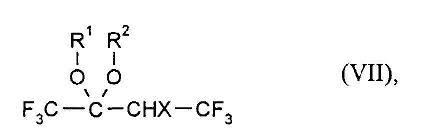 in welcher
- R¹, R² und X: die bei Formel (I') angegebenen Bedeutungen haben,
mit Hydroxyverbindungen der Formel (VI) in Gegenwart eines Säurebindemittels umsetzt.

Dies ist überraschend, weil in J. Indian Chem. Soc. 30, 809 (1953) beschrieben ist, daß man bei der Umsetzung von 1,1,1,4,4,4-Hexafluor-2,3-dichlor-2-buten mit methanolischer KOH neben 1,1,1,4,4,4-Hexafluor-3-chlor-2,2-dimethoxybuten, welches eine Verbindung der Formel (VII) darstellt, 1,4,4,4-Tetrafluor-3-chlor-1,1,2-trimethoxybutan erhält, welches bei Erhöhung der Temperatur zum bevorzugten Produkt wird.

Die Verbindungen der Formel (VII) sind neu, ausgenommen 1,1,1,4,4,4-Hexafluor-2,2-dimethoxy-3-chlorbutan, 1,1,1,4,4,4-Hexafluor-2,2-diethoxy-3-chlorbutan, 1,1,1,4,4,4-Hexafluor-2,2-ethylendioxy-3-chlorbutan und 1,1,1,2,4,4,4-Heptafluor-2,2-ethylendioxybutan.

Weiterhin wurde gefunden, daß sich die Verbindungen der Formel (VII) in einem Verfahren (C) herstellen lassen, das dadurch gekennzeichnet ist, daß man in einem ersten Schritt Hexafluorbutene der Formel (VIII) 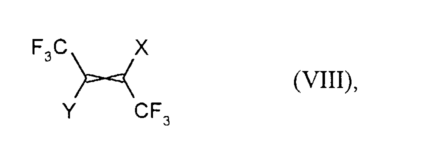 in welcher
- X: die bei Formel (I') angegebene Bedeutung hat,
- Y: für Fluor, Chlor oder Brom steht und
die gekreuzte Doppelbindung für die E- und/oder Z-Form steht,
mit Alkoholen der Formeln (IX) und (X)

R¹―OH (IX)

R²―OH (X),

in denen
- R¹ und R²: die bei Formel (I') angegebene Bedeutung haben,
in Gegenwart eines Säurebindemittels umsetzt und gegebenenfalls in einem zweiten Schritt mit einem anderen Alkohol der Formel (X) in Gegenwart eines Säurebindemittels umsetzt.

In einer bevorzugten Ausführungsform des Verfahrens (A) erhält man Verbindungen der Formel (I), indem man die Reaktionsmischung aus dem Verfahren (B) mit Säure versetzt.

In einer bevorzugten Ausführungsform des Verfahrens (B) erhält man Verbindungen der Formel (IV) oder (V) ausgehend von Hexafluorbutenen der Formel (VIII), indem man die Reaktionsbedingungen bei Verfahren (C) so wählt, daß die zunächst entstehende Verbindung der Formel (VII) direkt weiterreagiert.

In einer ganz besonderen Ausführungsform kombiniert man alle drei Verfahren im o.a Sinne, so daß man "in einem Topf" Hexafluorbutene der Formel (VIII) zu speziellen 3,3-Dioxy-4,4,4-trifluorbuttersäure-Derivaten der Formel (I), in welchen R¹, R² und R³ jeweils für einen identischen Rest stehen umsetzt. Dieses besonders einfache und kostengünstige Verfahren ist insbesondere in Hinblick auf die Verwendung der Verbindungen der Formel (I) zur Herstellung der Trifluoracetone der Formel (II) und der Trifluoracetessigester der Formel (III) vorteilhaft.

Die Verbindungen der Formeln (I), (III), (IV), (V) und (VII) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sind Gegenstand der vorliegenden Erfindung.

Die neuen 3,3-Dioxy-4,4,4-trifluorbuttersäure-derivate sind durch die allgemeine Formel (I) definiert.
- R¹ und R²: stehen bevorzugt unabhängig voneinander für C₁-C₆-Alkyl, insbesondere Methyl, Ethyl, n-Propyl, Isopropyl, n-Butyl, Isobutyl, sec.-Butyl oder tert.-Butyl, für Cyclohexyl, für gegebenenfalls durch Halogen, Nitro, Cyano, C₁-C₄-Alkyl, Trifluormethyl, C₁-C₄-Alkoxy, Trifluormethoxy, Difluormethoxy oder Amino substituiertes Phenyl, Naphthyl, Benzyl oder Phenethyl.
- R¹ und R²: stehen bevorzugt auch gemeinsam für gegebenenfalls ein- oder zweifach durch Methyl substituiertes C₂-C₄-Alkylen, insbesondere Ethylen oder für gegebenenfalls durch Halogen, Nitro, Cyano, C₁-C₄-Alkyl, Trifluormethyl, C₁-C₄-Alkoxy, Trifluormethoxy, Difluormethoxy oder Amino substituiertes ortho-Phenylen, o-Naphtylen oder o-Anthrylen.
- R³: steht bevorzugt für Wasserstoff, C₁-C₆-Alkyl, insbesondere Methyl, Ethyl, n-Propyl, Isopropyl, n-Butyl, Isobutyl, sec.-Butyl oder tert.-Butyl, für Cyclohexyl, gegebenenfalls durch Halogen, Nitro, Cyano, C₁-C₄-Alkyl, Trifluormethyl, C₁-C₄-Alkoxy, Trifluormethoxy, Difluormethoxy oder Amino substituiertes Phenyl, Naphthyl, Benzyl oder Phenethyl.

Aus dem bevorzugten Bereich ausgenommen sind 4,4,4-Trifluor-3,3-dimethoxybuttersäuremethylester, 4,4,4-Trifluor-3,3-dimethoxybuttersäure, 4,4,4-Trifluor-3,3-diethoxybuttersäureethylester, 4,4,4-Trifluor-3,3-ethylendioxybuttersäureethylester und 4,4,4-Trifluor-3,3-ethylendioxybuttersäure.
- R¹ und R²: stehen besonders bevorzugt unabhängig voneinander für C₁-C₆-Alkyl, insbesondere Methyl, Ethyl, n-Propyl, Isopropyl, n-Butyl, Isobutyl, sec.-Butyl oder tert.-Butyl, für Cyclohexyl, für gegebenenfalls durch Fluor, Chlor, Brom, Nitro, Cyano, C₁-C₄-Alkyl, Trifluormethyl, C₁-C₄-Alkoxy, Trifluormethoxy, Difluormethoxy oder Amino substituiertes Phenyl, Naphthyl oder Benzyl.
- R¹ und R²: stehen besonders bevorzugt auch gemeinsam für gegebenenfalls ein-oder zweifach durch Methyl substituiertes C₂-C₃-Alkylen, insbesondere Ethylen oder für gegebenenfalls durch Fluor, Chlor, Brom, Nitro, Cyano, C₁-C₄-Alkyl, Trifluormethyl, C₁-C₄-Alkoxy, Trifluormethoxy, Difluormethoxy oder Amino substituiertes ortho-Phenylen, oder o-Naphtylen.

Aus dem besonders bevorzugten Bereich ausgenommen sind 4,4,4-Trifluor-3,3-dimethoxybuttersäuremethylester, 4,4,4-Trifluor-3,3-dimethoxybuttersäure, 4,4,4-Trifluor-3,3 -diethoxybuttersäureethyl ester, 4,4,4-Trifluor-3,3-ethylendi oxybuttersäureethylester und 4,4,4-Trifluor-3,3-ethylendioxybuttersäure.
- R¹ und R²: stehen ganz besonders bevorzugt unabhängig voneinander für Methyl, Ethyl, n-Propyl, Isopropyl, n-Butyl, Isobutyl, sec.-Butyl oder tert.-Butyl, für Cyclohexyl, für gegebenenfalls durch Fluor, Chlor, Brom, Nitro, Cyano, Methyl, Ethyl, Propyl, Isopropyl, Trifluormethyl, Methoxy, Ethoxy, Trifluormethoxy, Difluormethoxy oder Amino substituiertes Phenyl oder Benzyl.
- R¹ und R²: stehen ganz besonders bevorzugt auch gemeinsam für Ethylen, 1,2-Propylen, 1,3-Propylen, 2,4-Butylen oder für gegebenenfalls durch Fluor, Chlor, Brom, Nitro, Cyano, Methyl, Ethyl, Propyl, Isopropyl, Trifluormethyl, Methoxy, Ethoxy, Trifluormethoxy, Difluormethoxy oder Amino substituiertes ortho-Phenylen, oder o-Naphtylen.
- R³: steht ganz besonders bevorzugt für Wasserstoff, Methyl, Ethyl, n-Propyl, Isopropyl, n-Butyl, Isobutyl, sec.-Butyl oder tert.-Butyl, für Cyclohexyl, für gegebenenfalls durch Fluor, Chlor, Brom, Nitro, Cyano, Methyl, Ethyl, Propyl, Isopropyl, Trifluormethyl, Methoxy, Ethoxy, Trifluormethoxy, Difluormethoxy oder Amino substituiertes Phenyl oder Benzyl.

Aus dem ganz besonders bevorzugten Bereich ausgenommen sind 4,4,4-Trifluor-3,3-dimethoxybuttersäuremethylester, 4,4,4-Trifluor-3,3-dimethoxybuttersäure, 4,4,4-Trifluor-3,3 -diethoxybuttersäureethylester, 4,4,4-Trifluor-3,3 -ethylendioxybuttersäureethylester und 4,4,4-Trifluor-3,3-ethylendioxybuttersäure.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein und, soweit möglich, z.B. ein- bis dreimal vorhanden sein können.

Verwendet man beispielsweise 1,4,4,4-Tetrafluor-2-chlor-1-ethoxy-3,3-dimethoxy-1-buten und Ethanol als Ausgangsstoffe, so kann der Reaktionsablauf des erfindungsgemäßen Verfahrens (A) durch das folgende Formelschema beispielhaft wiedergegeben werden:

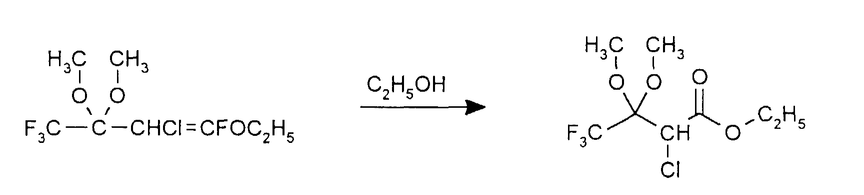

Verwendet man beispielsweise 1,1,1,3,4,4,4-Heptafluor-2,2-diethoxybutan und Methanol als Ausgangsstoffe, so kann der Reaktionsablauf des erfindungsgemäßen Verfahrens (B) durch das folgende Formelschema beispielsweise wiedergegeben werden:

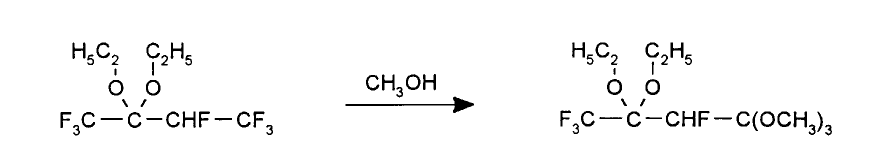

Verwendet man beispielsweise E-1,1,1,2,4,4,4-Heptafluor-3-chlor-2-buten und Ethandiol als Ausgangsstoffe, so kann der Reaktionsablauf des erfindungsgemäßen Verfahrens (C) durch das folgende Formelschema beispielsweise wiedergegeben werden:

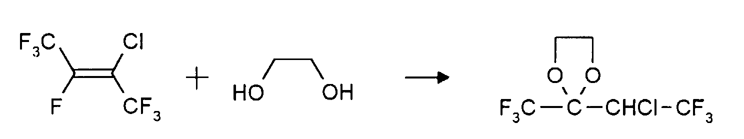

Die zur Durchführung des erfindungsgemäßen Verfahrens (A) benötigten Ketale sind durch die Formeln (IV) und (V) allgemein definiert. In diesen Formeln stehen R¹, R², R³ und X vorzugsweise für diejenigen Reste, die bereits im Zusammenhang mit der Beschreibung der 3,3-Dioxy-4,4,4-trifluorbuttersäure-Derivate der Formel (I) als bevorzugte Substituenten genannt wurden. Die Indizes n, m, o und p stehen auch bevorzugt wie oben angegeben. Es können sowohl reine Verbindungen der Formel (IV) oder der Formel (V), Gemische verschiedener Verbindungen der Formel (IV) mit denselben Resten, aber unterschiedlichen n und m für sich sowie verschiedener Verbindungen der Formel (V) mit denselben Resten, aber unterschiedlichen o und p als auch Gemische der Verbindungen der Formeln (IV) und (V) mit denselben Resten in beliebigem Verhältnis zu einander eingesetzt werden.

Die Verbindungen der Formeln (IV) und (V) sind neu und können z.B. nach Verfahren (B) hergestellt werden.

Die zur Durchführung des erfindungsgemäßen Verfahrens (A) benötigten Hydroxyverbindungen sind durch die Formel (VI) allgemein definiert. In dieser Formel steht R³ vorzugsweise für solche Reste, die bereits im Zusammenhang mit der Beschreibung der 3,3-Dioxy-4,4,4-trifluorbuttersäure-Derivate der Formel (I) als bevorzugte Substituenten genannt wurde.

Die Verbindungen der Formel (VI) sind allgemein bekannte chemische Verbindungen.

Das erfindungsgemäße Verfahren (A) wird in Gegenwart eines Protonenspenders durchgeführt. Als Protonenspender kommen alle anorganischen und organischen Protonen- wie auch Lewissäuren (wegen der Gegenwart einer Hydroxyverbindung), sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, saure Ionenaustauscher, saure Tonerden und saures Kieselgel.

Das Verfahren (A) wird vorzugsweise in Gegenwart eines Verdünnungsmittel durchgeführt. Hierfür kommen besonders bevorzugt Wasser und/oder ein Überschuß der Hydroxyverbindung (VI), daneben organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan.

Die Reaktionstemperaturen können bei der Durchführung des erfindungsgemäßen Verfahrens (A) in einem größeren Bereich variiert werden. Im allgemeinen arbeitet man bei Temperaturen zwischen 0 °C und +250 °C, vorzugsweise bei Temperaturen zwischen +20 °C und +150 °C.

Das Verfahren (A) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (A) setzt man pro Mol Ketal der Formel (IV) und/oder (V) beispielsweise 0,8 bis 100 Mol, vorzugsweise 1 bis 20 Mol Hydroxyverbindung der Formel (VI) und 0,1 bis 100 Mol, vorzugsweise 1 bis 50 Mol Protonenspender zu.

Die Reaktionsdurchführung, Aufarbeitung und Isolierung der Reaktionsprodukte kann nach allgemein üblichen, bekannten Verfahren erfolgen (vergl. auch die Beispiele).

Die zur Durchführung des erfindungsgemäßen Verfahrens (B) benötigten Dioxyhexafluorbutane sind durch die Formel (VII) allgemein definiert. In dieser Formel stehen R¹, R² und X vorzugsweise für diejenigen Reste, die bereits im Zusammenhang mit der Beschreibung der 3,3-Dioxy-4,4,4-trifluorbuttersäure-Derivate der Formel (I) als bevorzugte Substituenten genannt wurden.

Die Verbindungen der Formel (VII) sind bis auf die drei o.a. Ausnahmen neu und können z.B. nach Verfahren (C) hergestellt werden.

Die zur Durchführung des erfindungsgemäßen Verfahrens (B) benötigten Hydroxyverbindungen sind durch die Formel (VI) allgemein definiert und bei Verfahren (A) beschrieben.

Das erfindungsgemäße Verfahren (B) wird in Gegenwart eines geeigneten Säureakzeptors durchgeführt. Als solche kommen alle üblichen anorganischen und organischen Basen infrage. Hierzu gehören vorzugsweise Erdalkalimetall- und Alkalimetallhydride, -hydroxide, -amide, -alkoholate, -acetate, -carbonate und -hydrogencarbonate, wie beispielsweise Natriumhydrid, Natriumamid, Natrium-methylat, Natrium-ethylat, Kalium-tert.-butylat, Natriumhydroxid, Kalium- und Ammoniumhydroxid, Natrium-, Kalium-, Calcium- und Ammoniumacetat, Natrium-, Kalium-und Ammoniumcarbonat, Natriumhydrogen- und Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) und Diazabicycloundecen (DBU). Besonders bevorzugt setzt man ein Alkalimetallsalz der jeweils eingesetzten Hydroxyverbindung der Formel (VI) ein.

Das Verfahren (B) kann gegebenenfalls in Gegenwart eines Verdünnungsmittel durchgeführt werden. Hierfür kommen Wasser, ein Überschuß der Hydroxyverbindung (VI), organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan.

Das erfindungsgemäße Verfahren (B) kann gegebenenfalls in Gegenwart eines geeigneten Phasentransferkatalysators durchgeführt werden. Als Beispiele für solche Katalysatoren seien genannt: Tetrabutylammoniumiodid, -bromid und -chlorid, Tributylmethylphosphoniumbromid, Trimethyl-C₁₃-C₁₅-alkylammoniumchlorid und -bromid, Dibenzyldimethylammonium-methylsulfat, Dimethyl-C₁₂-C₁₄-alkylbenzylammoniumchlorid, 15-Krone-5, 18-Krone-6 oder Tris-[2-(2-methoxyethoxy)-ethyl]amin,

Die Reaktionstemperaturen können bei der Durchführung des erfindungsgemäßen Verfahrens (B) in einem größeren Bereich variiert werden. Im allgemeinen arbeitet man bei Temperaturen zwischen 0 °C und +250 °C, vorzugsweise bei Temperaturen zwischen +20 °C und +150 °C.

Das Verfahren (B) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (B) setzt man pro Mol Dioxyhexafluorbutan der Formel (VII) beispielsweise 1 bis 1000 Mol, vorzugsweise 1,5 bis 500 Mol Hydroxyverbindung der Formel (VI) und 1 bis 100 Mol, vorzugsweise 1,2 bis 20 Mol Säureakzeptor zu.

Die Reaktionsdurchführung, Aufarbeitung und Isolierung der Reaktionsprodukte kann nach allgemein üblichen, bekannten Verfahren erfolgen (vergl. auch die Beispiele).

Die zur Durchführung des erfindungsgemäßen Verfahrens (C) benötigten Hexafluorbutene sind durch die Formel (VIII) allgemein definiert. In dieser Formel steht Y vorzugsweise für Fluor oder Chlor.

Die Verbindungen der Formel (VIII) sind aus EP-315 783 A1 bekannt und zum Teil kommerziell erhältlich.

Die zur Durchführung des erfindungsgemäßen Verfahrens (C) benötigten Alkohole sind durch die Formel (IX) und gegebenenfalls die Formel (X) allgemein definiert. In diesen Formeln stehen R¹ und R² vorzugsweise für diejenigen Reste, die bereits im Zusammenhang mit der Beschreibung der 3,3-Dioxy-4,4,4-trifluorbuttersäure-Derivate der Formel (I) als bevorzugte Substituenten genannt wurden. Bei Einsatz monofunktionaler Alkohole können ein Alkohol der Formel (IX) alleine oder in Mischung mit einem anderen der Formel (X) eingesetzt werden. Vorzugsweise verwendet man nur einen Alkohol, dann ist formal Formel (IX) identisch mit Formel (X). Es können auch Diole eingesetzt werden, dann stehen R¹ und R² gemeinsam für einen der o.a. bifunktionalen Reste.

Die Verbindungen der Formeln (IX) und (X) sind allgemein bekannte chemische Verbindungen.

Das erfindungsgemäße Verfahren (C) wird in Gegenwart eines geeigneten Säureakzeptors durchgeführt. Als solche kommen alle üblichen anorganischen und organischen Basen infrage. Hierzu gehören vorzugsweise Erdalkalimetall- und Alkalimetallhydride, -hydroxide, -amide, -alkoholate, -acetate, -carbonate und -hydrogencarbonate, wie beispielsweise Natriumhydrid, Natriumamid, Natrium-methylat, Natrium-ethylat, Kalium-tert.-butylat, Natriumhydroxid, Kalium- und Ammoniumhydroxid, Natrium-, Kalium-, Calcium- und Ammoniumacetat, Natrium-, Kalium-und Ammoniumcarbonat, Natriumhydrogen- und Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) und Diazabicycloundecen (DBU). Besonders bevorzugt setzt man ein Alkalimetallsalz der jeweils eingesetzten Hydroxyverbindung der Formel (VI) ein.

Das Verfahren (C) kann gegebenenfalls in Gegenwart eines Verdünnungsmittel durchgeführt werden. Hierfür kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie Formamid, N,N-Dimethylformamid, N,N-Dimethylacetamid, N-Methylformanilid, N-Methylpyrrolidon oder Hexamethylphosphorsäuretriamid; N-Oxide wie N-Methylmorpholin-N-oxid; Ester wie Methyl-, Ethyl- oder Butylacetat; Sulfoxide, wie Dimethylsulfoxid; Sulfone, wie Sulfolan.

Das erfindungsgemäße Verfahren (C) kann gegebenenfalls in Gegenwart eines geeigneten Phasentransferkatalysators durchgeführt werden. Man kann beispielsweise die bei Verfahren (B) aufgelisteten Katalysatoren verwenden.

Die Reaktionstemperaturen können bei der Durchführung des erfindungsgemäßen Verfahrens (C) in einem größeren Bereich variiert werden. Im allgemeinen arbeitet man bei Temperaturen zwischen -80 °C und +150 °C, vorzugsweise bei Temperaturen zwischen 0 °C und +120 °C.

Das Verfahren (C) wird üblicherweise unter Normaldruck durchgeführt. Es ist jedoch auch möglich, unter erhöhtem oder vermindertem Druck zu arbeiten.

Zur Durchführung des erfindungsgemäßen Verfahrens (C) setzt man pro Mol Hexafluorbuten der Formel (VIII) beispielsweise je 1 bis 100 Mol, vorzugsweise 1,5 bis 50 Mol Alkohol(e) der Formeln (IX) und (X) und 1 bis 100 Mol, vorzugsweise 1,2 bis 20 Mol Säureakzeptor zu.

Die Reaktionsdurchführung, Aufarbeitung und Isolierung der Reaktionsprodukte kann nach allgemein üblichen, bekannten Verfahren erfolgen (vergl. auch die Beispiele).

### Beispiele

### Beispiel 1

### 2-Chlor-4,4,4-trifluor-3,3-dimethoxybutancarbonsäuremethylester

Zu 200 g 2,3-Dichlor-1,1,1,4,4,4-hexafluorbuten-2 wurden 618 g einer 30%-igen Lösung von Natriummethylat in Methanol zugetropft und 16 h unter Rückfluß erhitzt. Die Reaktionsmischung wurde auf Raumtemperatur abgekühlt und mit 150 ml 50%ige Schwefelsäure versetzt. Anschließend wurde wieder 3 h bei Rückflußtemperatur gerührt. Die Mischung wurde mit Wasser versetzt und mit Methylenchlorid extrahiert. Nach Trocknung über Natriumsulfat und Entfernung des Lösungsmittels wurde im Vakuum destilliert (Kp : 85°C / 10 mbar).
Ausbeute: 178 g 2-Chlor-4,4,4-trifluor-3,3-dimethoxybutancarbonsäuremethylester.
¹H-NMR (CDCl₃) δ [ppm]: 3,5 (s, 3H); 3,6 (s, 3H); 3.83 (s, 3H); 4,68 (s, 1H)
¹⁹F-NMR (CDCl₃) δ [ppm]: -76.18 (s)

### Beispiel 2

### Umsetzung von 2,3-Dichlor-1,1,1,4,4,4-hexafluorbuten-2 mit Natriummethylat

Zu 400 g 2,3-Dichlor-1,1,1,4,4,4-hexafluorbuten-2 in 200 ml Methanol wurden 1280 g einer 30%-igen Lösung von Natriummethylat in Methanol zugetropft und 10 h unter Rückfluß erhitzt. Die ausgefallenen Salze wurden abfiltriert und das Methanol wurde im Vakuum entfernt. Der Rückstand wurde mit Wasser versetzt und mit MTBE extrahiert. Nach Trocknung der organischen Phase mit Natriumsulfat und Entfernung des Lösungsmittels erhielt man 452 g einer Mischung aus 38 % 1,1,1,4,4-Pentafluor-3-chlor-2,2,4-trimethoxybutan, 23 % 2-Chlor-1,4,4,4-tetrafluor-1,3,3-trimethoxy-buten-1 und 31 % 2-Chlor-4,4,4-trifluor-3,3-dimethoxybutancarbonsäuremethylester.

### Beispiel 3

### 2-Chlor-4,4,4-trifluor-3,3-dimethoxybutancarbonsäuremethylester

300 g der Reaktionsmischung aus Beispiel 2 in 800 ml Methanol wurden mit 150 ml 50%-iger Schwefelsäure versetzt und 5 h unter Rückfluß gerührt. Das Lösungsmittel wurde weitgehend abrotiert und der Rest auf Wasser gegossen. Die Mischung wurde mit Methylenchlorid extrahiert, über Natriumsulfat getrocknet und das Lösungsmittel im Vakuum entfernt. Nach Destillation (Kp: 85°C/ 10 mbar) erhielt man 271 g 2-Chlor-4,4,4-trifluor-3,3-dimethoxybutancarbonsäuremethylester.

### Beispiel 4

### 2-Chlor-4,4,4-trifluor-3,3-dimethoxybutancarbonsäuremethylester

100 g der Reaktionsmischung aus Beispiel 2 wurden mit 500 ml 50%-iger Schwefelsäure versetzt und 5 h bei 80°C gerührt. Die Mischung wurde mit Methylenchlorid extrahiert, über Natriumsulfat getrocknet und das Lösungsmittel im Vakuum entfernt. Nach Destillation erhielt man 73 g 2-Chlor-4,4,4-trifluor-3,3-dimethoxybutancarbonsäuremethylester.

### Beispiel 5

### 2-Chlor-1,4,4,4-tetrafluor-1,3,3-triethoxybuten-1

Zu 233 g 2,3-Dichlor-1,1,1,4,4,4-hexafluorbuten-2 in 300 ml Ethanol wurden 1400 ml einer 20%-igen Lösung von Natriumethylat in Ethanol zugetropft und 2 h unter Rückfluß erhitzt. Die ausgefallenen Salze wurden abfiltriert und das Ethanol im Vakuum entfernt. Der Rückstand wurde mit Wasser versetzt und mit MTBE extrahiert. Nach Trocknung der organischen Phase mit Natriumsulfat und Entfernung des Lösungsmittels erhielt man 229 g 2-Chlor-1,4,4,4-tetrafluor-1,3,3-triethoxy-buten-1.
¹H-NMR (CDCl₃) δ [ppm]: 1,3 (m, 9H); 3,7 (m, 6H); 4,2 (m, 2H)
¹⁹F-NMR (CDCl₃) δ [ppm]: -78,2 (d); -81 (q)

### Beispiel 6

### 2-Chlor-4,4,4-trifluor-3,3-diethoxybutancarbonsäureethylester

100 g 2-Chlor-1,4,4,4-tetrafluor-1,3,3-triethoxy-buten-1 in 200 ml Ethanol wurden mit 100 ml 50%-ige Schwefelsäure versetzt und 3 h bei Rückfluß gerührt. Das Gemisch wurde auf Wasser gegossen und mit Methylenchlorid extrahiert. Die organische Phase wurde über Natriumsulfat getrocknet und das Lösungsmittel im Vakuum entfernt. Der Rückstand wurde im Vakuum destilliert. Es wurden 79 g 2-Chlor-4,4,4-trifluor-3,3-diethoxybutancarbonsäureethylester mit einem Siedepunkt 48°C bei 0,2 mbar isoliert.
¹H-NMR (CDCl₃) δ [ppm]: 1,3 (m, 9H); 3,67 (m, 1H); 3,72 (m, 1H); 3,82 (m, 1H); 4,09 (m, 1H); 4,25 (m, 2H); 4,63 (s, 1H)
¹⁹F-NMR (CDCl₃) δ [ppm]: -76.95 (s)

### Beispiel 7

### 2-Chlor-4,4,4-trifluor-3,3-dimethoxybutancarbonsäureethylester

Zu 100 g 2-Chlor-1,1,1,4,4,4-hexafluor-3,3-dimethoxy-butan in 50 ml Ethanol wurden 265 g 20%-ige Natriumethylatlösung in Ethanol bei Raumtemperatur zugetropft. Anschließend wurde 12 h bei Rückfluß gerührt, die Reaktionsmischung auf Raumtemperatur abgekühlt und 100 ml 50%-ige Schwefelsäure zugetropft. Nach nochmaligem 3-stündigem Rückfluß wurde die Mischung auf Wasser gegossen, mit Methylenchlorid extrahiert und die organische Phase wurde über Natriumsulfat getrocknet. Nach Entfernung des Lösungsmittels wurde im Vakuum destilliert.
Ausbeute: 82 g (Kp : 89°C/14 mbar)
¹H-NMR (CDCl₃) δ [ppm]: 1,3 (t, 3H); 3.5 (s, 3H), 3,58 (s, 3H); 4,28 (q, 2H); 4,62 (s, 1H)
¹⁹F-NMR (CDCl₃) δ [ppm]: -76,03 (s)

### Beispiel 8

### 2-Chlor-4,4,4-trifluor-3,3-dimethoxybutancarbonsäure

50 g 2-Chlor-1,1,1,4,4,4-hexafluor-3,3-dimethoxy-butan, 0,5 g Tetrabutylammoniumbromid und 300 ml Wasser wurden mit 32 g Natriumhydroxyd versetzt und 12 h bei 100 °C gerührt. Unter Kühlung wurden 50 ml 50%-ige Schwefelsäure zugetropft und anschließend die Reaktionsmischung auf 100 ml Wasser gegossen. Die Mischung wurde zweimal mit 200 ml Methylenchlorid extrahiert und die vereinigten organischen Phasen über Natriumsulfat getrocknet. Nach Entfernung des Lösungsmittels im Vakuum erhielt man 42 g 2-Chlor-4,4,4-trifluor-3,3-dimethoxybutancarbonsäure.
¹H-NMR (CDCl₃) δ [ppm]: 3,52 (s, 3H); 3,59 (s, 3H); 4,68 (s, 1H); 9.5 (s, 1H)
¹⁹F-NMR (CDCl₃) δ [ppm]: -75.7 (s)

### Beispiel 9

### 2-Chlor-1,1,1,4,4,4-hexafluor-3,3-ethylendioxybutan

Zu 400 ml Toluol, 80 g Ethylenglylol und 80 g 60%-iges Natriumhydrid in Mineralöl wurden 233 g 2,3-Dichlor-1,1,1,4,4,4-hexafluorbuten-2 bei Raumtemperatur zugetropft. Es wurde 5 h bei 40°C gerührt und anschließend die Reaktionsmischung mit Wasser versetzt. Die organische Phase wurde abgetrennt, die wäßrige Phase mit MTBE extrahiert und die vereinigten die organischen Phasen über Natriumsulfat getrocknet. Nach Entfernung des Lösungsmittels und anschließender Destillation im Vakuum erhielt man 180 g 2-Chlor-1,1,1,4,4,4-hexafluor-3,3-ethylendioxybutan (Kp 48 °C / 16 mbar).
¹H-NMR (CDCl₃) δ [ppm]: 4,28 (m, 4H); 4,5 (q, 1H)
¹⁹F-NMR (CDCl₃) δ [ppm]: -68,75 (m); -79,9 (q)

### Beispiel 10

### 2-Chlor-4,4,4-trifluor-3,3-ethylendioxybutancarbonsäureethylester

30 g 2-Chlor-1,1,1,4,4,4-hexafluor-3,3-ethylendioxybutan in 30 ml Ethanol wurden mit 80 g 20%-ige Natriumethylatlösung in Ethanol versetzt und 10 h unter Rückfluß gerührt. Anschließend wurden 100 ml 50%ige Schwefelsäure zudosiert und 6 h unter Rückfluß gerührt. Die Mischung wurde auf Eis gegossen und mit Methylenchlorid extrahiert. Nach Trocknung über Natriumsulfat und Entfernung des Lösungsmittels und anschließender Destillation im Vakuum erhielt man 24 g 2-Chlor-4,4,4-trifluor-3,3-ethylendioxybutancarbonsäureethylester mit einem Siedepunkt von 108°C / 20 mbar.
¹H-NMR (CDCl₃) δ [ppm]:1,3 (t, 3H), 4,28 (m, 6H), 4,62 (s, 1H)
¹⁹F-NMR (CDCl₃) δ [ppm]: -80,2 (s)

### Beispiel 11

### 1-Chlor-4,4,4-trifluoraceton

Zu 400 ml konz. Schwefelsäure bei 140 °C wurden 250 g 2-Chlor-4,4,4-trifluor-3,3-dimethoxybutancarbonsäuremethylester in 2 zugetropft und das Produkt gleichzeitig abdestilliert. Das Destillat wurde anschließend über eine 10 cm Vigreuxkolonne redestilliert. Man erhielt 122 g 1-Chlor-3,3,3-trifluoraceton (Ausbeute 83%) mit einem Siedepunkt von 73 °C.

## Patentansprüche

1. Verbindungen der Formel (I) 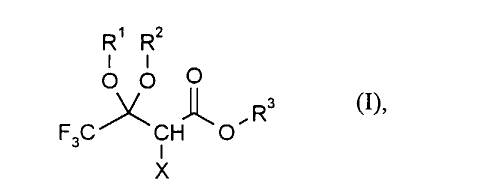 in welcher
R¹ und R² unabhängig voneinander für C₁-C₁₀-Alkyl, für Cyclohexyl, für gegebenenfalls substituiertes C₆-C₁₀ -Aryl oder C₇-C₁₄-Arylalkyl stehen oder
R¹ und R² gemeinsam für gegebenenfalls ein- oder zweifach durch Methyl substituiertes C₂-C₄-Alkylen oder gegebenenfalls substituiertes ortho-Arylen stehen,
R³ für Wasserstoff, C₁-C₁₀-Alkyl, für Cycloalkyl, für gegebenenfalls substituiertes C₆-C₁₀-Aryl oder C₇-C₁₄-Arylalkyl steht und
X für Wasserstoff, Fluor, Chlor oder Brom steht,
wobei 4,4,4-Trifluor-3,3-dimethoxybuttersäuremethylester, 4,4,4-Trifluor-3,3-dimethoxybuttersäure, 4,4,4-Trifluor-3,3-diethoxybuttersäureethylester, 4,4,4-Trifluor-3,3-ethylendioxybuttersäureethylester und 4,4,4-Trifluor-3,3-ethylendioxybuttersäure ausgenommen sind.

2. Verwendung von Verbindungen des Anspruchs 1 zur Herstellung der entsprechenden 4,4,4-Trifluoracetessigesterderivate der Formel 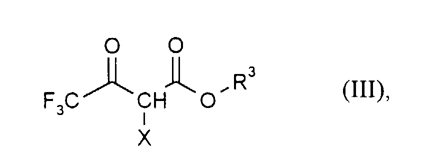 in der
R³ und X die in Anspruch 1 angegebene Bedeutung haben,
durch Einwirkung von Lewis-Säuren.

3. Verfahren zur Herstellung von Verbindungen der Formel 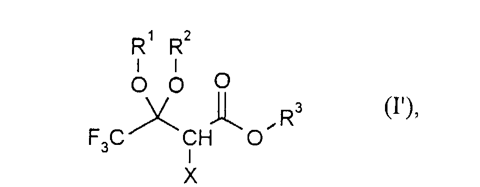 in welcher
R¹ und R² unabhängig voneinander für C₁-C₁₀-Alkyl, für Cyclohexyl, für gegebenenfalls substituiertes C₆-C₁₀ -Aryl oder C₇-C₁₄-Arylalkyl stehen oder
R¹ und R² gemeinsam für gegebenenfalls ein- oder zweifach durch Methyl substituiertes C₂-C₄-Alkylen oder gegebenenfalls substituiertes ortho-Arylen stehen,
R³ für Wasserstoff, C₁-C₁₀-Alkyl, für Cyclohexyl, für gegebenenfalls substituiertes C₆-C₁₀-Aryl oder C₇-C₁₄-Arylalkyl steht und
X für Wasserstoff, Fluor, Chlor oder Brom steht,
dadurch gekennzeichnet, daß man Verbindungen der Formel (IV) oder (V) 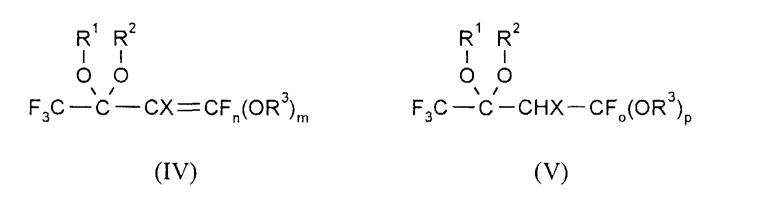 in welchen
R¹, R², R³ und X die oben bei Formel (I') angegebene Bedeutung haben und
n und o unabhängig voneinander für die Zahlen 0, 1 oder 2 stehen,
m für die Zahlen 0, 1 oder 2 steht, wobei n+m = 2 ist und
p für 1, 2 oder 3 steht, wobei o+p = 3 ist,
mit einer Hydroxyverbindung der Formel (VI)
R³―OH (VI),
in der
R³ die bei den Formeln (IV) und (V) angegebene Bedeutung hat,
in Gegenwart eines Protonenspenders umsetzt.

4. Verfahren zur Herstellung von Verbindungen der in Anspruch 3 angegebenen Formeln (IV) und (V), dadurch gekennzeichnet, daß man Dioxyhexafluorbutane der Formel 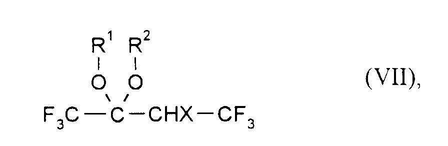 in welcher
R¹, R² und X die oben bei Formel (I') angegebenen Bedeutungen haben,
mit Hydroxyverbindungen der Formel
R³-OH (VI),
in der
R³ die in Anspruch 3 bei Formel (I') angegebene Bedeutung hat,
in Gegenwart eines Säurebindemittels umsetzt.

5. Verfahren zur Herstellung von Verbindungen der Formel 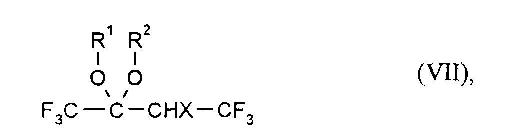 in der
R¹, R² und X die in Anspruch 3 bei Formel (I') angegebene Bedeutung haben,
dadurch gekennzeichnet, daß man in einem ersten Schritt Hexafluorbutene der Formel 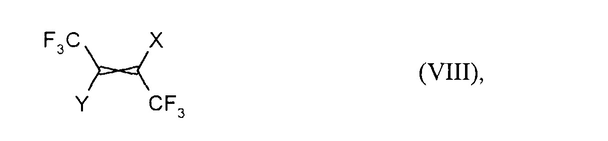 in der
X die in Anspruch 3 bei Formel (I') angegebene Bedeutung hat,
Y für Fluor, Chlor oder Brom steht und
die gekreuzte Doppelbindung für die E- und/oder Z-Form steht,
mit Alkoholen der Formeln
R¹-OH (IX)
und
R²-OH (X),
in denen
R¹ und R² die in Anspruch 3 bei Formel (I') angegebene Bedeutung haben,
in Gegenwart eines Säurebindemittels umsetzt.

6. Verfahren nach Anspruch 5, dadurch gekennzeichnet, daß man zusätzlich in einem zweiten Schritt mit einem anderen Alkohol der Formel (X) in Gegenwart eines Säurebindemittels umsetzt.

7. Verfahren zur Herstellung von Verbindungen der Formel (I') (siehe Anspruch 3), bei denen R¹, R² und R³ identisch sind, dadurch gekennzeichnet, daß man die Verfahren der Ansprüche 5, gegebenenfalls 6, 4 und 3 in einem Topf kombiniert.

8. Verwendung von Verbindungen des Anspruchs 1 zur Herstellung von 1,1,1-Trifluoracetonen der Formel 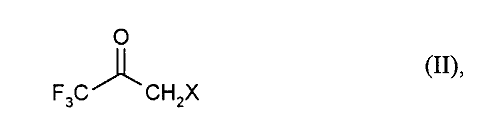 in der
X die in Anspruch 1 angegebene Bedeutung hat,
durch Einwirkung von Mineralsäuren.

9. Verbindungen der Formeln 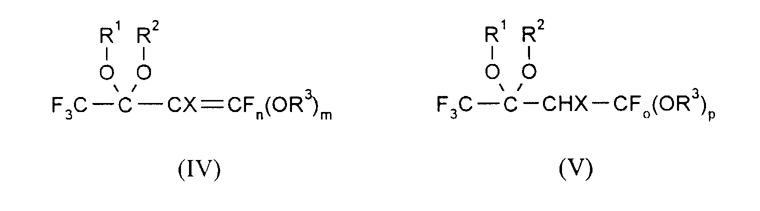 in welchen
R¹ und R² unabhängig voneinander für C₁-C₁₀-Alkyl, für Cyclohexyl, für gegebenenfalls substituiertes C₆-C₁₀ -Aryl oder C₇-C₁₄-Arylalkyl stehen oder
R¹ und R² gemeinsam für gegebenenfalls ein- oder zweifach durch Methyl substituiertes C₂-C₄-Alkylen oder gegebenenfalls substituiertes ortho-Arylen stehen,
R³ für Wasserstoff, C₁-C₁₀-Alkyl, für Cyclohexyl, für gegebenenfalls substituiertes C₆-C₁₀-Aryl oder C₇-C₁₄-Alylalkyl steht und
X für Wasserstoff, Fluor, Chlor oder Brom steht,
n und o unabhängig voneinander für die Zahlen 0, 1 oder 2 stehen,
m für die Zahlen 0, 1 oder 2 steht, wobei n+m = 2 ist und
p für 1, 2 oder 3 steht, wobei o+p = 3 ist.

10. Verbindungen der Formel 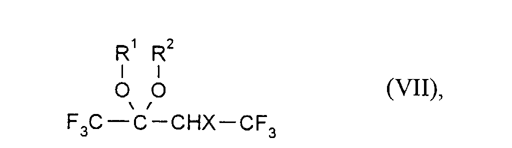 in welcher
R¹, R² und X die in Anspruch 1 angegebene Bedeutung haben,
ausgenommen 1,1,1,4,4,4-Hexafluor-2,2-dimethoxy-3-chlorbutan, 1,1,1 ,4,4,4-Hexafluor-2,2-diethoxy-3-chlorbutan, 1,1,1,4,4,4-Hexafluor-2,2-ethylendioxy-3-chlorbutan und 1,1,1,2,4,4,4-Heptafluor-2,2-ethylendioxybutan.

## Claims

1. Compound of the formula (I) 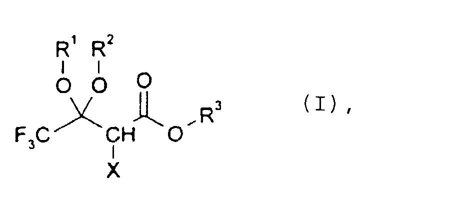 in which
R¹ and R² independently of one another represent C₁-C₁₀-alkyl, or represent cyclohexyl, or represent optionally substituted C₆-C₁₀-aryl or C₇-C₁₄-arylalkyl, or
R¹ and R² together represent C₂-C₄-alkylene which is optionally mono- or disubstituted by methyl, or optionally substituted ortho-arylene,
R³ represents hydrogen or C₁-C₁₀-alkyl, or represents cycloalkyl, or represents optionally substituted C₆-C₁₀-aryl or C₁-C₁₄-arylalkyl, and
X represents hydrogen, fluorine, chlorine or bromine,
excluding methyl 4,4,4-trifluoro-3,3-dimethoxybutyrate, 4,4,4-trifluoro-3,3-dimethoxybutyric acid, ethyl 4,4,4-trifluoro-3,3,-diethoxybutyrate, ethyl 4,4,4-trifluoro-3,3-ethylenedioxybutyrate and 4,4,4-trifluoro-3,3-ethylenedioxybutyric acid.

2. Use of a compound of Claim 1 for the preparation of the corresponding 4,4,4-trifluoroacetoacetate derivative of the formula 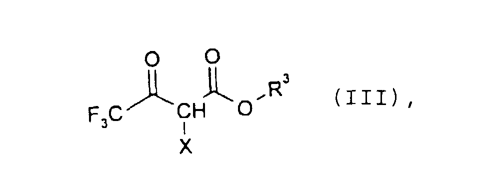 in which
R³ and X have the meaning given in Claim 1,
by the action of a Lewis acid.

3. Process for the preparation of a compound of the formula 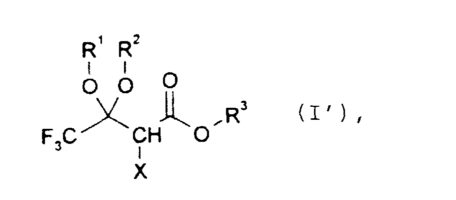 in which
R¹ and R² independently of one another represent C₁-C₁₀-alkyl, or represent cyclohexyl, or represent optionally substituted C₆-C₁₀-aryl or C₁-C₁₄-arylalkyl, or
R¹ and R² together represent C₂-C₄-alkylene which is optionally mono- or disubstituted by methyl, or optionally substituted ortho-arylene,
R³ represents hydrogen or C₁-C₁₀-alkyl, or represents cyclohexyl, or represents optionally substituted C₆-C₁₀-aryl or C₇-C₁₄-arylalkyl, and
X represents hydrogen, fluorine, chlorine or bromine,
characterized in that a compound of the formula (IV) or (V) 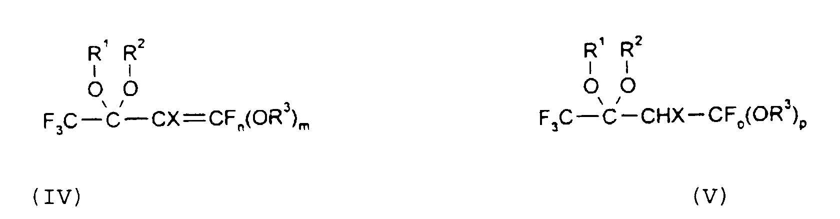 in which
R¹, R², R³ and X have the meaning given above in the case of formula (I') and
n and o independently of one another represent the numbers 0, 1 or 2,
m represents the numbers 0, 1 or 2, where n+m = 2 and
p represents 1, 2 or 3, where o+p = 3,
is reacted with a hydroxy compound of the formula (VI)
R³―OH (VI),
in which
R³ has the meaning given in the case of the formulae (IV) and (V),
in the presence of a proton donor.

4. Process for the preparation of a compound of the formula (IV) or (V) given in Claim 3, characterized in that a dioxyhexafluorobutane of the formula 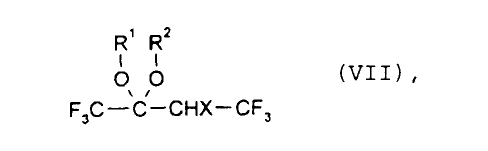 in which
R¹, R² and X have the meanings given above in the case of formula (I'),
is reacted with a hydroxy compound of the formula
R³-OH (VI),
in which
R³ has the meaning given in the case of formula (I') in Claim 3,
in the presence of an acid-binding agent.

5. Process for the preparation of a compound of the formula 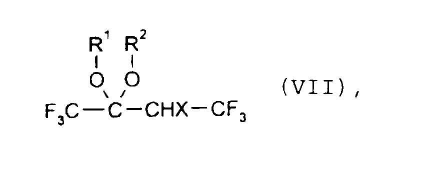 in which
R¹, R² and X have the meaning given in the case of formula (I') in Claim 3,
characterized in that, in a first step, a hexafluorobutene of the formula 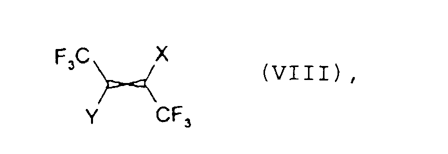 in which
X has the meaning given in the case of formula (I') in Claim 3,
Y represents fluorine, chlorine or bromine and
the crossed double bond represents the E and/or Z form,
is reacted with an alcohol of the formula
R¹-OH (IX)
or
R²-OH (X),
in which
R¹ and R² have the meaning given in the case of formula (I') in Claim 3,
in the presence of an acid-binding agent.

6. Process according to Claim 5, characterized in that, in a second step, the product is additionally reacted with another alcohol of the formula (X) in the presence of an acid-binding agent.

7. Process for the preparation of a compound of the formula (I') (see Claim 3), in which R¹, R² and R³ are identical, characterized in that the processes of Claims 5, if appropriate 6, 4 and 3, are combined in one pot.

8. Use of a compound of Claim 1 for the preparation of a 1,1,1-trifluoroacetone of the formula 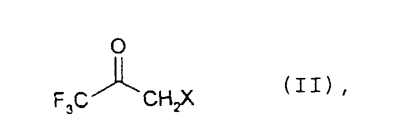 in which
X has the meaning given in Claim 1,
by the action of a mineral acid.

9. Compound of the formula 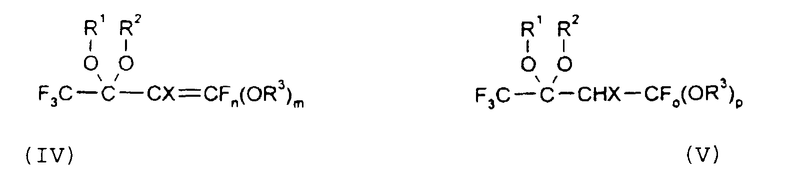 in which
R¹ and R² independently of one another represent C₁-C₁₀-alkyl, or represent cyclohexyl, or represent optionally substituted C₆-C₁₀-aryl or C₁-C₁₄-arylalkyl, or
R¹ and R² together represent C₂-C₄-alkylene which is optionally mono- or disubstituted by methyl, or optionally substituted ortho-arylene,
R³ represents hydrogen or C₁-C₁₀-alkyl, or represents cycloalkyl, or represents optionally substituted C₆-C₁₀-aryl or C₇-C₁₄-arylalkyl, and
X represents hydrogen, fluorine, chlorine or bromine,
n and o independently of one another represent the numbers 0, 1 or 2,
m represents the numbers 0, 1 or 2, where n+m = 2 and
p represents 1, 2 or 3, where o+p = 3.

10. Compound of the formula 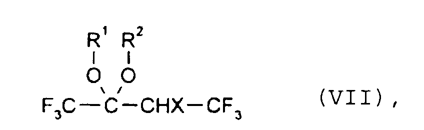 in which
R¹, R² and X have the meaning given in Claim 1,
excluding 1,1,1,4,4,4-hexafluoro-2,2-dimethoxy-3-chlorobutane, 1,1,1,4,4,4-hexafluoro-2,2-diethoxy-3-chlorobutane, 1,1,1,4,4,4-hexafluoro-2,2-ethylenedioxy-3-chlorobutane and 1,1,1,2,4,4,4-heptafluoro-2,2-ethylenedioxy- butane.

## Revendications

1. Composés répondant à la formule (I) 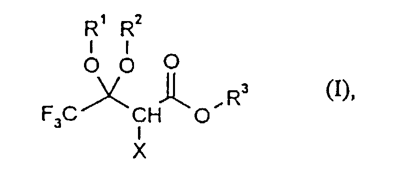 dans laquelle
R¹ et R² représentent, indépendamment l'un de l'autre, un groupe alkyle en C₁-C₁₀; un groupe cyclohexyle; un groupe aryle en C₆-C₁₀ ou un groupe arylalkyle en C₇-C₁₄ le cas échéant substitué, ou
R¹ et R² représentent ensemble un groupe alkylène en C₂-C₄ portant le cas échéant un ou deux substituants méthyle ou représentent un groupe ortho-arylène le cas échéant substitué,
R³ représente un atome d'hydrogène, un groupe alkyle en C₁-C₁₀; un groupe cycloalkyle; un groupe aryle en C₆-C₁₀ ou un groupe arylalkyle en C₇-C₁₄ le cas échéant substitué, et
X représente un atome d'hydrogène, un atome de fluor, un atome de chlore ou un atome de brome,
l'ester méthylique de l'acide 4,4,4-trifluoro-3,3-diméthoxybutyrique, l'acide 4,4,4-trifluoro-3,3-diméthoxybutyrique, l'ester éthylique de l'acide 4,4,4-trifluoro-3,3-diéthoxybutyrique, l'ester éthylique de l'acide 4,4,4-trifluoro-3,3-éthylène-dioxybutyrique et l'acide 4,4,4-trifluoro-3,3-éthylènedioxybutyrique étant exclus.

2. Utilisation de composés selon la revendication 1 pour la préparation des dérivés esters 4,4,4-trifluoro-acétylacétiques correspondants répondant à la formule 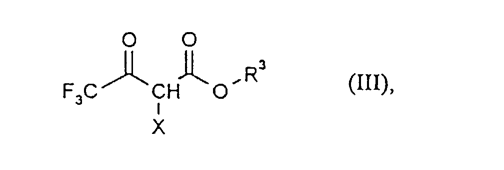 dans laquelle
R³ et X ont la signification indiquée à la revendication 1,
sous l'action d'acides de Lewis.

3. Procédé pour la préparation de composés répondant à la formule 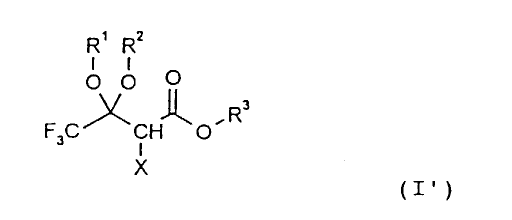 dans laquelle
R¹ et R² représentent, indépendamment l'un de l'autre, un groupe alkyle en C₁-C₁₀; un groupe cyclohexyle; un groupe aryle en C₆-C₁₀ ou un groupe arylalkyle en C₇-C₁₄ le cas échéant substitué, ou
R¹ et R² représentent ensemble un groupe alkylène en C₂-C₄ portant le cas échéant un ou deux substituants méthyle ou représentent un groupe ortho-arylène le cas échéant substitué,
R³ représente un atome d'hydrogène, un groupe alkyle en C₁-C₁₀; un groupe cyclohexyle; un groupe aryle en C₆-C₁₀ ou un groupe arylalkyle en C₇-C₁₄ le cas échéant substitué, et
X représente un atome d'hydrogène, un atome de fluor, un atome de chlore ou un atome de brome,
caractérisé en ce qu'on fait réagir des composés répondant à la formule (IV) ou à la formule (V) 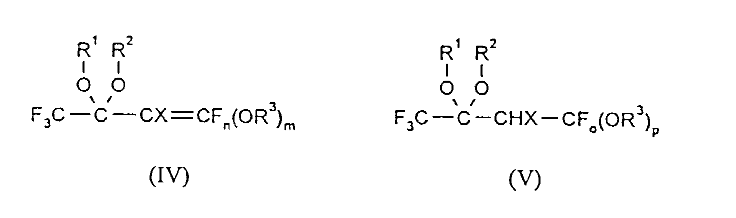 dans lesquelles
R¹, R², R³ et X ont la signification indiquée ci-dessus dans le cadre de la formule (I') et
n et o représentent, indépendamment l'un de l'autre, les nombres 0, 1 ou 2,
m représente les nombres 0, 1 ou 2, n+m étant égal à 2, et
p représente 1, 2 ou 3, o+p étant égal à 3,
avec un composé hydroxylé répondant à la formule (VI)
R³-OH (VI)
dans laquelle
R³ a la signification indiquée dans les formules (IV) et (V),
en présence d'un agent cédant des protons.

4. Procédé pour la préparation de composés répondant aux formules (IV) ou (V) indiquées à la revendication 3, caractérisé en ce qu'on fait réagir des dioxyhexafluorobutanes répondant à la formule 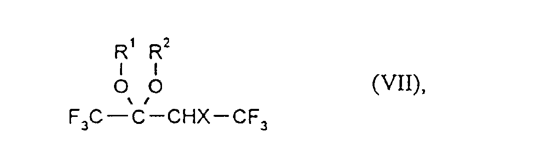 dans laquelle
R¹, R² et X ont la signification indiquée ci-dessus dans le cadre de la formule (I')
avec des composés hydroxylés répondant à la formule
R³-OH (VI)
dans laquelle
R³ a la signification indiquée à la revendication 3 dans le cadre de la formule (I'),
en présence d'un agent neutralisant les acides.

5. Procédé pour la préparation de composés répondant à la formule 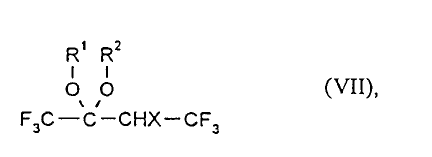 dans laquelle
R¹, R² et X ont la signification indiquée à la revendication 3 dans le cadre de la formule (I'),
caractérisé en ce qu'on fait réagir, dans une première étape, des hexafluorobutènes répondant à la formule 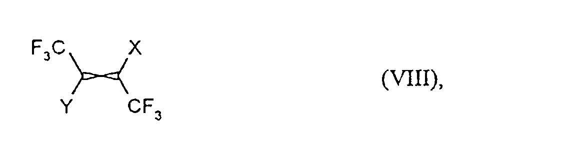 dans laquelle
X a la signification indiquée à la revendication 3 dans le cadre de la formule (I'),
Y représente un atome de fluor, un atome de chlore ou un atome de brome,
et la liaison double croisée représente la forme E et/ou la forme Z,
avec des alcools répondant aux formules
R¹-OH (IX)
et
R²-OH (X)
dans lesquelles
R¹ et R² ont la signification indiquée à la revendication 3 dans le cadre de la formule (I'),
en présence d'un agent neutralisant les acides.

6. Procédé selon la revendication 5, caractérisé en ce qu'on procède en outre à une mise en réaction dans une deuxième étape avec un autre alcool répondant à la formule (X) en présence d'un agent neutralisant les acides.

7. Procédé pour la préparation de composés répondant à la formule (I') (voir la revendication 3) dans lesquels R¹, R² et R³ sont identiques, caractérisé en ce qu'on combine les procédés des revendications 5, le cas échéant 6, 4 et 3 en un seul pot.

8. Utilisation de composés selon la revendication 1 pour la préparation de 1,1,1-trifluoro-acétones répondant à la formule 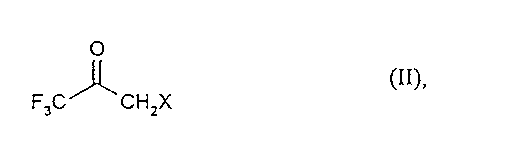 dans laquelle
X a la signification indiquée à la revendication 1,
sous l'action d'acides minéraux.

9. Composés répondant aux formules 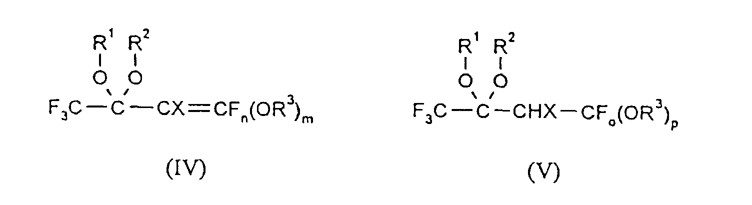 dans lesquelles
R¹ et R² représentent, indépendamment l'un de l'autre, un groupe alkyle en C₁-C₁₀; un groupe cyclohexyle; un groupe aryle en C₆-C₁₀ ou un groupe arylalkyle en C₇-C₁₄ le cas échéant substitué, ou
R¹ et R² représentent ensemble un groupe alkylène en C₂-C₄ portant le cas échéant un ou deux substituants méthyle ou représentent un groupe ortho-arylène le cas échéant substitué,
R³ représente un atome d'hydrogène, un groupe alkyle en C₁-C₁₀; un groupe cyclohexyle; un groupe aryle en C₆-C₁₀ ou un groupe arylalkyle en C₇-C₁₄ le cas échéant substitué, et
X représente un atome d'hydrogène, un atome de fluor, un atome de chlore ou un atome de brome,
n et o représentent, indépendamment l'un de l'autre, les nombres 0, 1 ou 2,
m représente les nombres 0, 1 ou 2, n+m étant égal à 2, et
p représente 1, 2 ou 3, o+p étant égal à 3.

10. Composés répondant à la formule 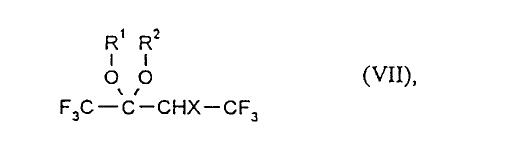 dans laquelle
R¹, R² et X ont la signification indiquée à la revendication 1,
à l'exclusion du 1,1,1,4,4,4-hexafluoro-2,2-diméthoxy-3-chlorobutane, du 1,1,1,4,4,4-hexafluoro-2,2-diéthoxy-3-chlorobutane, du 1,1,1,4,4,4-hexafluoro-2,2-éthylènedioxy-3-chlorobutane et du 1,1,1,2,4,4,4-heptafluoro-2,2-éthylènedioxybutane.
